# EUROPEAN PATENT SPECIFICATION

(11) **EP 0 470 310 B1**
(45) Date of publication and mention of the grant of the patent: **29.11.1995**
(21) Application number: 90308787.2
(22) Date of filing: 09.08.1990
(51) Int. Cl.: C07D 311/60, A61K 31/35

(54) **Novel benzopyrans and process for their production**
Benzopyrane und Verfahren zu deren Herstellung
Benzopyrannes et procédé pour leur production

(43) Date of publication of application: 12.02.1992
(73) Proprietor: Council of Scientific and Industrial Research, New Delhi 110 001 (IN)
(72) Inventor: Kapil, Randir Singh Regional Research Laboratory, State of Jammu & Kashmir (IN); Durani, Susheel Central Drug Research Institute, Uttar Pradesh (IN); Dhar, Janak Dulari Central Drug Research Institute, Uttar Pradesh (IN); Setty, Bachu Sreenivasulu, Uttar Pradesh (IN)
(74) Representative: Sexton, Jane Helen

(56) References cited:
- US-A- 3 314 975

## Description

The present invention relates to novel benzopyrans which are anti-estrogenics and possess significant activity against implantation and breast cancer. The invention also relates to a process for the synthesis of these benzopyrans.

A number of anti-estrogens have been synthesised in recent years. These non-steroidal compounds have been developed on the basis of their structure activity relationship established by Dodds and his co-workers and have been found to be more potent than compounds known hereto (Dodds, E.C., J.Pharm. Pharmacol., 1, 137 (1949), Dodds, E.C., Folley, S.J., Glascuck, R.F. and Lawson, W., Biochem.J., 68, 161 (1958); Dodds, E.C., Goldberg, L., Lawson, W. and Robinson, R., Part I Proc. Royal Soc. B., 127, 140 (1939).

These anti-estrogens consist essentially of diarylethylenes (DAE) and triarylethylenes (TAE) and examples of the more important of such compounds in terms of potency include tamoxifen, 4-hydroxytamoxifen, centchroman and hydroxycentchroman.

Tamoxifen and 4-hydroxytamoxifen can be represented by the formula: 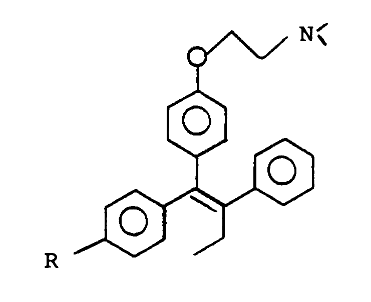
wherein when R is hydrogen the compound is tamoxifen and when R is OH the compound is 4-hydroxytamoxifen.

Centchroman and hydroxycentchroman can be represented by the formula: 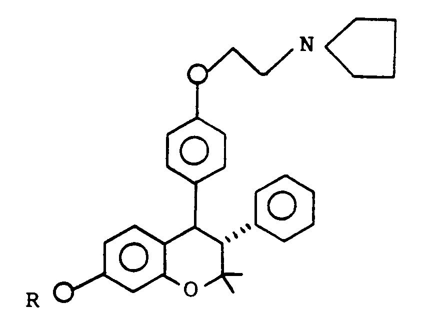
wherein when R is Me the compound is centchroman and when R is H the compound is hydroxycentchroman.

Unfortunately, almost all hitherto known anti-estrogens, including DAE's and TAE's evince varying degrees of agonistic activity. This is a drawback in their use as drugs since it is the estrogen antagonistic activity of DAE's and TAE's which makes them important.

It has therefore long been desired to develop novel estrogen antagonists which have extremely low, preferably essentially no agonistic activity, viz. which do not evince estrogenecity.

A study of known DAE's and TAE's has established that it is the presence of glyceryl ether or w-alkylaminoalkoxy chain in the para position in one of the geminal aryl groups that is essential for estrogen antagonistic activity. Two or three methylene groups and the nitrogen atom substituted with one or two short alkyl or single cycloalkyl residue (piperidino or pyrrolidino) in the said chain is the optimum requirement for estrogen antagonistic activity of the prototypes. Among TAEs, molecular geometry plays an important role in determining an agonist/antagonist balance. In most compounds, estrogen antagonist activity is restricted to only those isomers having two of the vicinal aryl groups, without the basic ether chain, in trans geometry to each other. The corresponding cis isomers are estrogens devoid of antagonistic activity.

The present invention therefore provides a compound of the formula I 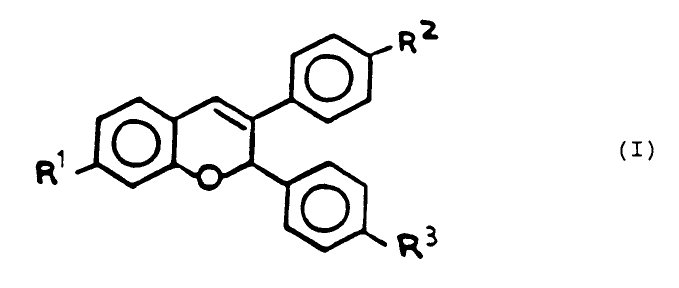
wherein R¹ and R², which may be the same or different, are each -H, -OH, alkoxy of 1 to 17 carbon atoms or alkoxycarbonyl of 2 to 18 carbon atoms, and R³ is

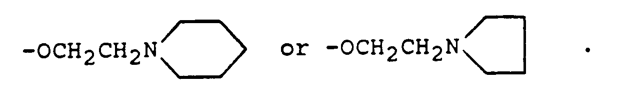

There are several preferred embodiments. In one preferred embodiment R¹ and R² are each independently H, OH or C₁₋₄ alkoxy.

Other preferred embodiments include (i) R¹ being H or (ii) R¹ and R² each being an acyl or alkoxy group.

R³ is preferably a 2-piperidinoethoxy group. Specific compounds within formula I include 2-[4-[2-(1-piperidino)ethoxy]phenyl]-3-[4-hydroxyphenyl]-2H-1-benzopyran, 2-[4-[2-(1-piperidino)ethoxy]phenyl]-3-phenyl-7-methoxy-2H-1-benzopyran, 2-[4-[2-(1-piperidino) ethoxy]phenyl]-3-[4-hydroxyphenyl]-7-hydroxy-2H-1-benzopyran.

The present invention also provides a process for the synthesis of these benzopyrans of the formula: 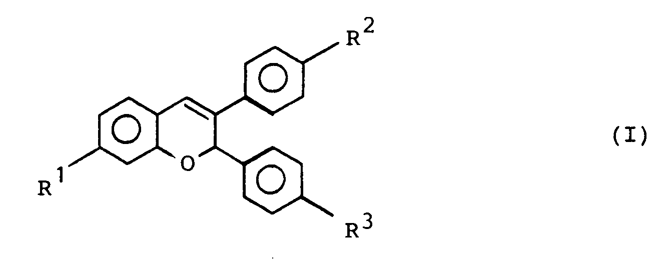
wherein R¹, R² and R³ have the meanings stated above. This process comprises reacting a compound of formula II 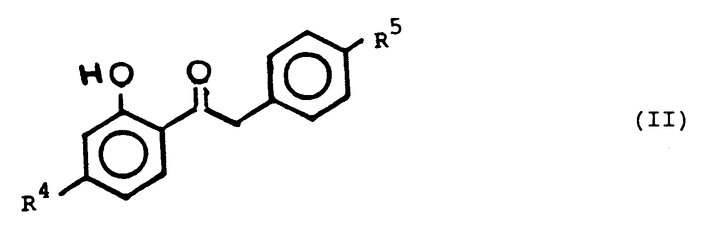
in which R⁴ and R⁵ are R¹ and R² respectively, or a protected hydroxy group, with 4-hydroxybenzaldehyde to produce a compound of the formula III 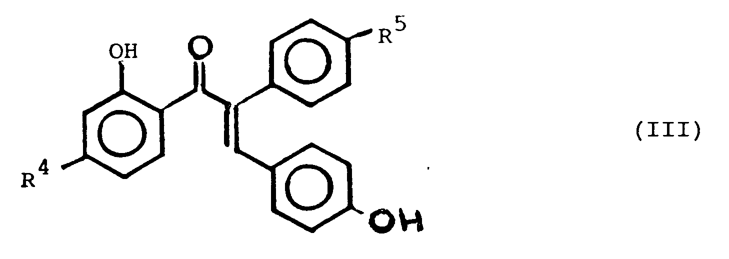
forming a compound of formula IV 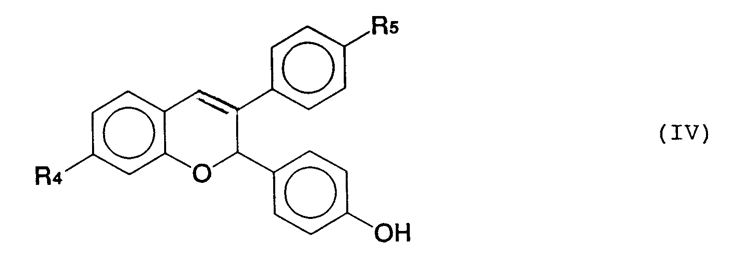
reacting this compound with a compound of formula V 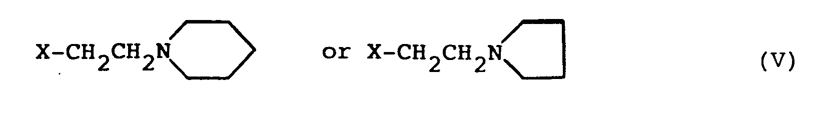
in which X is a halide to form a compound of formula VI 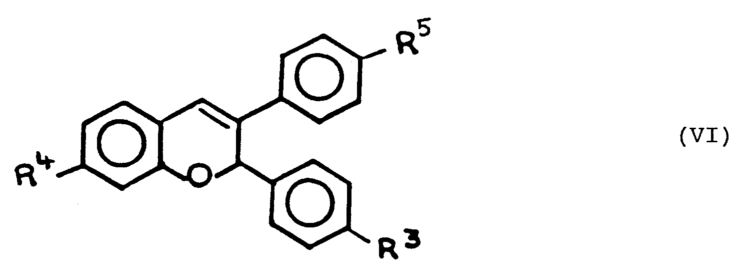
and, if necessary, deprotecting and acylating or alkylating R⁴ and R⁵.

In the preferred embodiment in which in the compound I produced, R¹ is H, preferably in the above process R⁴ is H and R⁵ is a protected hydroxy group.

When the process is used to produce a compound in which R¹ and R² are each alkoxy or carboxy then R⁴ and R⁵ may be R¹ and R² respectively or may each be in the form of a protected hydroxy group. If R¹ or R² is a hydroxy group then R⁴ or R⁵ respectively in the above process is preferably in the form of a protected hydroxy group. If R⁴ or R⁵ is a protected group then preferably the protecting group is 3,4-dihydropyran. The 3,4-dihydropyran may be reacted with a compound of formula IX 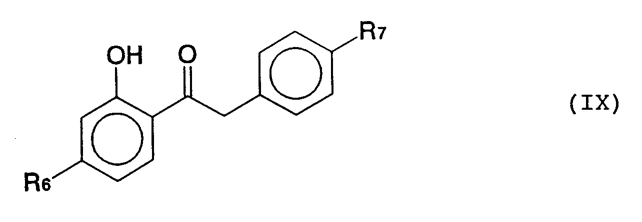
where one of R⁶ and R⁷ is a hydroxy group and the other is hydrogen or a hydroxy group or an alkoxy or carboxy group, to form a tetrahydropyranyl ether. Preferably the reaction is carried out in the presence of a sulphonic acid, such as para-toluene sulphonic acid or the like in an ether solvent, such as, dioxan or the like. The reaction may be effected for a period of up to 4 hours and the crude reaction product, after stipulated processing, may be purified e.g.by crystallization from a petroleum solvent such as hexane or by rapid chromatography over silica gel.

The reaction of the compound of formula II with the 4-hydroxybenzaldehyde may be effected in the presence of a cyclic or open chain secondary and/or tertiary amino base such as piperidine or triethyl amine, and an aromatic hydrocarbon solvent such as benzene or the like. The solvent may be added at periodic intervals to replenish its loss during the reaction. This reaction may be effected for a period of about 30 hours. Thereafter, the reaction mixture may be cooled and washed with water, the organic layer separated, dried under Na₂SO₄ and concentrated. The solidified material may be filtered off, washed with a halogenated solvent such as chloroform, methylene dichloride or the like to give a compound of the formula III. Generally the compound III will be produced as a mixture with a compound of formula VIII 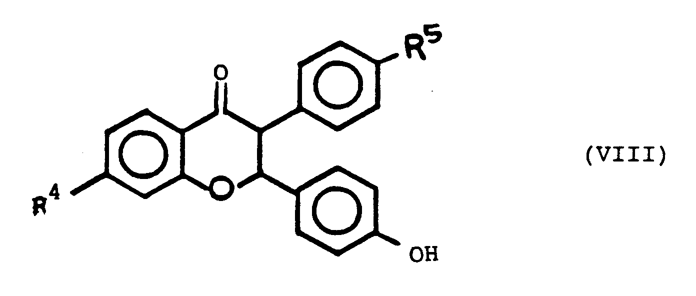
For example the product mixture may contain a ratio of compound VIII to compound III of 1.0:1.5. The filtrate containing compounds III and VIII may be concentrated, chromatographed and eluted with an eluate of increasing polarity such as ethyl acetate in hexane or the like, thereby separating out the compound of formula III.

The compound of the formula III may be converted to a compound of formula IV by reduction, for example by treating with a hydride such as sodium borohydride or the like in an alcoholic solvent such as ethyl alcohol or the like. Cyclodehydration may also be carried out, typically during work up of the product e.g. thermal work up may cause cyclodehydration. The hydride may be added in different proportions, at intervals of 10 to 15 minutes at room temperature under stirring. The reaction may be continued for a period of up to 12 to 15 hours. The reaction product after concentration, pH adjustment and extraction with a polar solvent such as ethyl acetate is purified by chromatography e.g. flash chromatography over silica gel to yield a compound of the formula IV. The compound of formula IV is treated with a piperidino- or pyrrolidinoalkyl halide, preferably in the presence of a basic catalyst such as potassium carbonate and a suitable ketonic solvent such as acetone or the like. This may be followed by purification by chromatography e.g. on alumina using hexane or a mixture thereof with a polar solvent to yield a compound of formula VI.

If R⁴ and R⁵ are not protected hydroxy groups then the compound produced is a compound of formula I. If R⁴ or R⁵ is a protected hydroxy group then the protecting group(s) may be removed by known methods e.g. by use of an acid such as hydrochloric acid in an alcoholic solvent such as ethanol. The deprotected hydroxy group(s) may, if desired, be alkylated or acylated by known methods to give other compounds of formula I.

The unprotected starting compound of formula (II)' 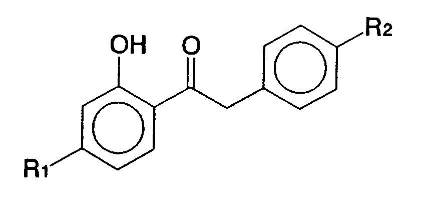
where R¹ and R² are as herein before defined can be prepared by methods known in the art. For example, when R¹ is H and R² is OH it can be prepared by condensation of phenol with 4-methoxy-phenylacetyl chloride (in turn prepared from 4-methoxy-phenylacetic acid) to afford an ester which on typical Fries rearrangement in the presence of anhydrous aluminium chloride yields a mixture which can be resolved chromatographically to afford the desired starting material. It can be characterised by its physical and spectral data.

When R¹ is e.g. methoxy and R² is as hereinbefore defined the starting compound II' may be prepared by Friedel-Crafts acylation of a corresponding phenol, such as 3-methoxyphenol or the like, with a suitable substituted or unsubstituted phenylacetyl chloride using a catalyst, such as anhydrous aluminium chloride. The resultant produce may be purified by steam distillation and/or column chromatography.

In turn, when R¹ and R² are both OH the starting compound can be prepared by Friedal-Crafts acylation of resorcinol with 4-methoxyphenyl acetyl chloride. This reaction affords a mixture of trihydroxydeoxybenzoin and methoxy dihydroxydeoxybenzoin. The latter compound may be converted into the desired trihydroxy compound by heating it with anhydrous pyridine hydrochloride.

The following examples illustrate the invention.

### EXAMPLE-AI

### PREPARATION OF MONOTETRAHYDROPYRANYL ETHER OF DIHYDROXYDEOXYBENZOIN (1-[2-HYDROXYPHENYL]-2-[4-(TETRAHYDROPYRAN-2-YL)OXY]PHENYL]ETHANONE)

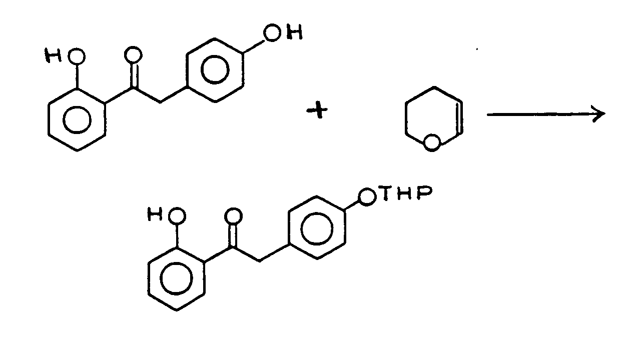

To a stirred solution of the deoxybenzoin (18.3 gm, 0.08 mole) and PTSA (100 mg) in dry dioxan (200 ml) was added dropwise a solution of 3,4-dihydro-2H-pyran (14.5 ml, 0.16 mole) in dry dioxan (100 ml) and the stirring was continued for 4 hours. The reaction mixture was then neutralised with methanolic ammonia (5 ml) and concentrated in vacuo. The residue was dissolved in ether (200 ml) and washed twice with 5% sodium hydroxide solution followed by water. The organic layer was dried (Na₂SO₄) and concentrated. The residue was chromatographed over a short silica gel column eluting rapidly with ethyl acetate-hexane (1:20, v/v)) to afford the title compound (20 gm, 80.1%) which was crystallised from hexane to m.p. 95°C.

### EXAMPLE-AII

### PREPARATION OF 1-[2-HYDROXYPHENYL]-2-[4-(TETRAHYDROPYRAN-2-YL)]OXY]PHENYL]-3-[4-HYDROXYPHENYL]PROP-2-ENONE

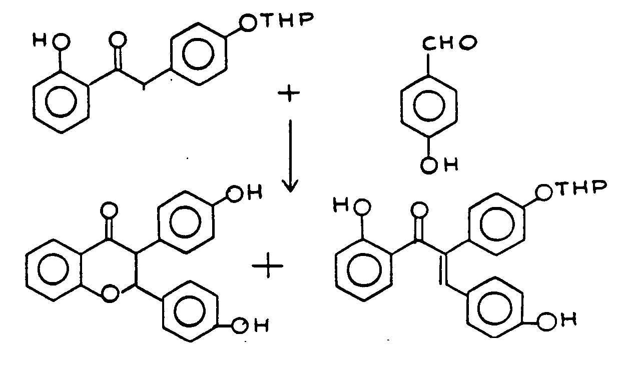

To a solution of the monotetrahydropyranyl ether of the dihydroxydeoxybenzoin (6.87 g, 22 mmole) and 4-hydroxybenzaldehyde (2.44 gm, 20 mmole) in dry benzene (100 ml) was added dry piperidine (0.12 ml). It was refluxed for 30 hours removing water azeotropically adding fresh portions of dry benzene from time to time to replenish the distilled benzene. The reaction mixture was cooled and washed with water (2 x 40 ml). The organic layer was dried (Na₂SO₄) and concentrated. The residue left was allowed to stand for 24 hours. The solidified material was filtered off and washed with chloroform to afford the dihydrobenzopyranone. The filtrate was collected, concentrated and residue chromatographed over a silica gel column eluting rapidly with 2% ethyl acetate in hexane, to afford, first, the unreacted THP ether of deoxybenzoin (starting compound). With increasing polarity (20% ethyl acetate in hexane), the 2-phenylchalcone (desired compound) was obtained which was crystallised from benzene-hexane, m.p. 140°C (6 g, 72.1%).

### EXAMPLE - AIII

### PREPARATION OF 2[4-HYDROXYPHENYL]-3-[4-[(TETRAHYDROPYRAN-2-YL]OXY] PHENYL]-2H-1-BENZOPYRAN

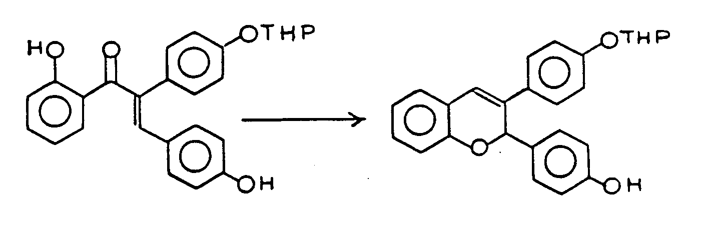

To a stirred solution of 2-phenylchalcone, prepared in example-AII, (4.16 gm, 10 mmole) in ethanol (50 ml) was added sodium borohydride (400 mg) in portions at an interval of 10-15 minutes and the stirring was continued for 15 hours. The ethanol was evaporated under vacuum and the residue was decomposed by adding saturated ammonium chloride solution until the pH was 8.0. The mixture was extracted with ethyl acetate. The organic layer was washed twice with water, dried and concentrated (Na₂SO₄). The residue was chromatographed over a silica gel column eluting with 10% ethyl acetate in hexane to afford the desired compound (2.4 gm, 60%) which was crystallised from benzene-hexane, m.p. 110°C.

### EXAMPLE-AIV

### 2-[4-[2-(1-PIPERIDINO)ETHOXY]PHENYL]-3-[4-HYDROXYPHENYL]-2H-1-BENZOPYRANS

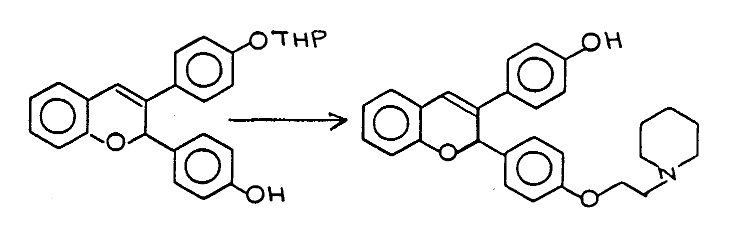

A mixture of the phenol prepared above in example AIII (800 mg, 2 mmole), 1-[2-chloroethyl] piperidine hydrochloride (590 mg, 3.2 mmole), anhydrous potassium carbonate (440 mg, 3.2 mmole) and dry acetone (20 ml) was stirred and refluxed for 35 hours. It was cooled and solid material filtered off and washed with acetone. The combined filtrate was concentrated and the residue chromatographed over a basic alumina column eluting with 2% ethyl acetate in hexane to afford the THP ether derivative (800 mg).

To a solution of this compound (800 mg) in ethanol (20 ml) was added 1 N hydrochloric acid (5 ml) and the solution was allowed to stand for 20 minutes. The ethanol was evaporated in vacuo, the residue treated with sodium bicarbonate solution (25 ml) and extracted twice with ethyl acetate. The organic layer was washed with water, dried (Na₂SO₄) and concentrated to afford the title compound of this invention (600 mg, 70.2%). It was crystallised from chloroformhexane, m.p. 110°C.

### EXAMPLE-BI

### PREPARATION OF 1-[2-HYDROXY-4-METHOXYPHENYL]-2-PHENYL-3-[4-HYDROXYPHENYL]PROP-2-ENONE

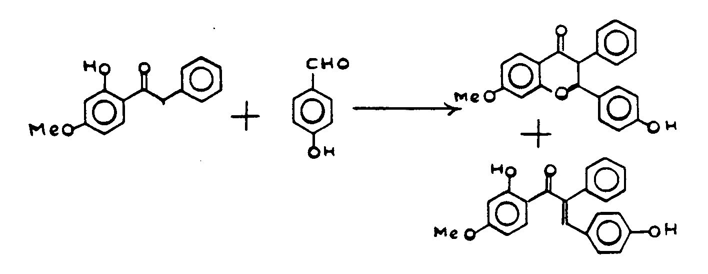

To a solution of deoxybenzoin i.e. 1-[2-hydroxy-4-methoxy-phenyl]-2-phenyl ethanone (12.1 g, 0.05 mole) and 4-hydroxy-benzaldehyde (6.1 g, 0.05 mole) in dry benzene (200 ml) was added dry piperidine (0.3 ml). The solution was refluxed for 30 hours removing water azeotropically and adding fresh proportions of dry benzene from time to time to replenish its loss during the reaction. It was then cooled and washed twice with water. The organic layer was dried (Na₂SO₄) and concentrated. The residue was allowed to stand for 24 hour, the solidified material filtered and washed with chloroform to afford the dihydrobenzopyranone (4.0 g, 23.2%). It was crystallised from ethyl acetate-hexane, m.p. 195°C.

The combined filtrate left after the removal of dihydrobenzopyranone was concentrated and the residue subjected to chromatography over a silica gel column eluting with ethyl acetate - hexane to afford, first, the unreacted deoxybenzoin (staring material). Then, on increasing the solvent polarity, the 2-phenyl chalcone i.e. compound desired in this example was obtained (11.0 g, 65.3%). It crystallised from benzene-hexane, m.p. 150°C.

### EXAMPLE-BII

### PREPARATION OF 2-[4-HYDROXYPHENYL]-3-PHENYL-7-METHOXY-2H-1-BENZOPYRAN

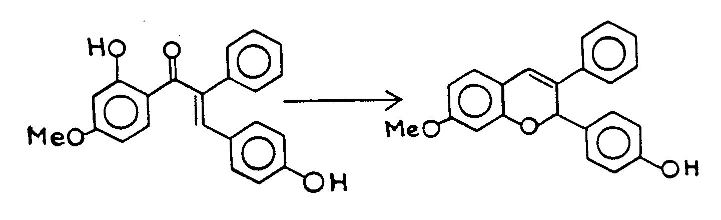

To a stirred solution of 2-phenylchalcone (prepared above in example-BI) (3.46 g, 10 mmole) in ethanol (15 ml) was added sodium borohydride (0.4 g) in three portions at 15 minute intervals and the stirring was continued for 12 hours. Ethanol was evaporated in vacuo and to the residue was added saturated ammonium chloride solution dropwise until the pH was 8.0. The mixture was extracted twice with ethyl acetate, the organic layer washed with water, dried (Na₂SO₄) and concentrated. The residue was chromatographed over a silica gel column eluting with ethyl acetate - hexane to afford the phenol desired under this example (2.5 g, 80%). It was crystallised from chloroform-hexane, m.p. 162°C.

### EXAMPLE-BIII

### PREPARATION OF 2-[4-[2-(1-PYRROLIDINO) ETHOXY]PHENYL]-3-PHENYL-7-METHOXY-2H-1-BENZOPYRAN

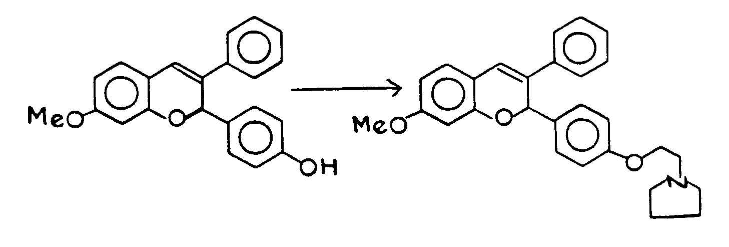

A mixture of the phenol prepared above in example-BII (660 mg, 2 mmole), 1-[2-chloro-ethyl] pyrrolidine hydrochloride (544 mg, 3.2 mmole), anhydrous potassium carbonate (440 mg, 3.2 mmole) and dry acetone (20 ml) was stirred and refluxed for 30 hours. On completion of the reaction (TLC), the reaction mixture was cooled, the solid material filtered off and washed with acetone. The combined filtrate was concentrated. The residue was chromatographed over a basic alumina column eluting with ethyl acetate - hexane to afford the pyrrolidino ether desired in this example (671 mg, 78.6%). It was crystallised from hexane, m.p. 99°C.

### EXAMPLE-BIV

### PREPARATION OF 2-[4-[2-(1-PIPERIDINO)ETHOXY]PHENYL]-3-PHENYL-7-METHOXY-2H-1-BENZOPYRAN

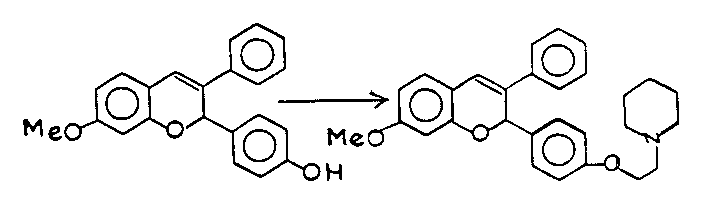

A mixture of phenol prepared above in example-BII (660 mg, 2 mmole), 1-[2-chloroethyl] piperidine hydrochloride (590 mg, 3.2 mmole), anhydrous potassium carbonate (440 mg, 3.2 mmole) and dry acetone (20 ml) was stirred and refluxed for 30 hours. On completion of the reaction (TLC), it was cooled, the solid material filtered off and washed with acetone. The combined filtrate was concentrated. The residue was chromatographed over a column of basic alumina eluting with ethyl acetate - hexane to afford the compound desired in this specification (717 mg, 81.5%).

### EXAMPLE-CI

### PREPARATION OF 1-[2-HYDROXY-4-[TETRAHYDROPYRAN-2-YL)OXY]PHENYL]-2-[4-[(TETRAHYDROPYRAN-2-YL) OXY]PHENYL]ETHANONE

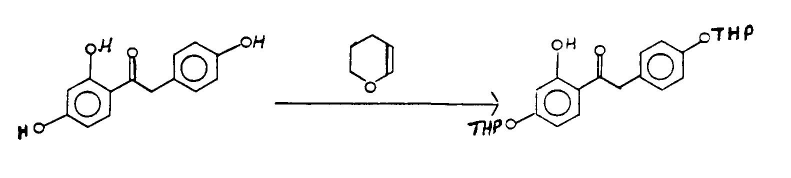

To the starting deoxybenzoin (2.44 g, 10 mmole) shown above, cooled in an ice bath, was added a mixture of 3,4-dihydro-2H-pyran (10 ml, 110 mmole) and concentrated hydrochloric acid (0.01 ml). The reaction mixture was stirred for 4 hours. It was then diluted with ether (100 ml) and washed thrice with 5% sodium hydroxide solution followed by water. The ethereal layer was dried (Na₂SO₄) and concentrated. The oily residue was crystallised from hexane to afford (3.87 g, 94% yield of the title compound, m.p. 118°C.

### EXAMPLE-CII

### PREPARATION OF 2-[4-HYDROXYPHENYL]-3-[4-[(TETRAHYDROPYRAN-2-YL)OXY]PHENYL]-7-[4-(TETRAHYDROPYRAN-2-YL)OXY]-2,3-DIHYDRO-4H-1-BENZOPYRAN-4-ONE AND 1-[2-HYDROXY-4-[(TETRAHYDROPYRAN-2-YL)OXY]PHENYL]-2-[4-[(TETRAHYDROPYRAN-2-YL)OXY]PHENYL]-3-[4-HYDROXYPHENYL]PROP-2-ENONE

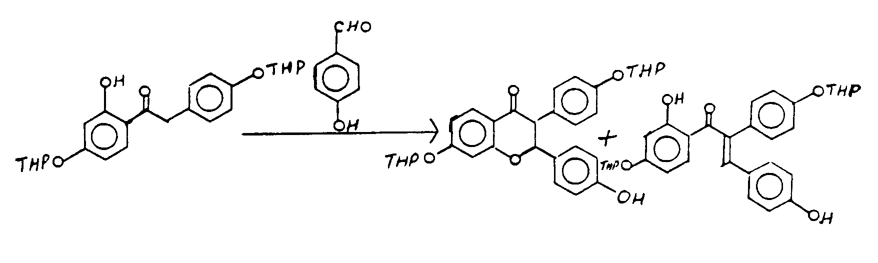

To a solution of bis (tetrahydropyranyl) ether of deoxybenzoin (4.12 g, 10 mmol), prepared above and 4-hydroxybenzaldehyde (1.22 g, 10 mmole) in dry benzene (100 ml) was added dry piperidine (0.01 ml). The reaction mixture was refluxed for 30 hours removing water azeotropically and adding dry benzene from time to time, if needed, to replenish loss during the reaction. The reaction mixture was cooled and washed twice with water. The organic layer was dried (Na₂SO₄) and concentrated. The residue was chromatographed over a silica gel column eluting with ethyl acetatehexane mixture to afford the unreacted deoxybenzoin ether. Then on increasing the solvent polarity a mixture of the dihydrobenzopyranone and 2-phenylchalcone was obtained. The dihydrobenzopyranone (1.2 g, 23%), m.p. 190°C, was separated by crystallisation from a mixture of ethyl acetate-hexane leaving behind the 2-phenylchalcone as an oily residue in the mother liquor. The 2-phenylchalcone (2.3 g, 44%) was used in the next step without purification.

### EXAMPLE-CIII

### PREPARATION OF 2-[HYDROXYPHENYL]-3-[4-[(TETRAHYDROPYRAN-2-YL)OXY]PHENYL]-7-[(TETRAHYDROPYRAN-2-YL)OXY]

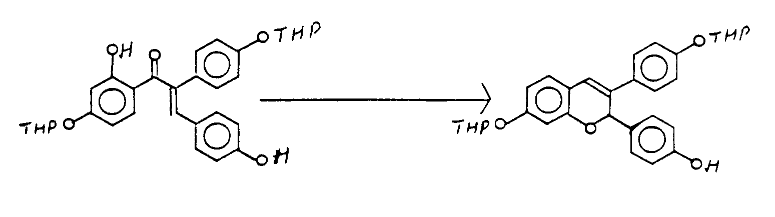

To a stirred solution of the 2-phenylchalcone (1.29 g, 2.5 mmole), prepared above in example-CII in ethyl alcohol (20 ml) was added sodium borohydride (100 mg) in different portions at an interval of 10-15 minutes at room temperature and the stirring was continued for 12 hours. Ethyl alcohol was evaporated in vacuo, and to the residue was added saturated ammonium chloride solution dropwise until the pH was 8.0. The mixture was extracted twice with ethyl acetate, the organic layer washed with water, dried (Na₂SO₄) and concentrated. The residue was purified by flash chromatography over a silica gel column eluting with ethyl acetate-hexane to afford the title phenol (940 mg, 75%), which was crystallised from chloroform-hexane, m.p. 180°C.

### EXAMPLE-CIV

### PREPARATION OF 2-[4-[2-(1-PIPERIDINO)ETHOXY] PHENYL]-3-[4-HYDROXYPHENYL]-7-HYDROXY-2H-1-BENZOPYRAN

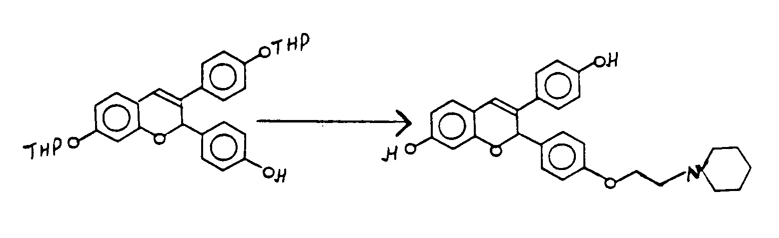

A mixture of the phenol (1.0 g, 2 mmole), prepared above in example-CIII, and 1-[2-chloroethyl] piperidine hydrochloride (590 mg, 3.2 mmole), anhydrous potassium carbonate (440 mg, 3.2 mmole) and dry acetone 30 ml was stirred and refluxed for 30 hours. On completion of the reaction (TLC), it was cooled, the solid material filtered and washed with acetone. The combined filtrate was concentrated and the residue chromatographed over a basic alumina column eluting with ethyl acetate-hexane to afford (1.1 g) of an oil residue. This was dissolved in ethyl alcohol (20 ml) and to it was added 1N hydrochloric acid (10 ml) and the solution was allowed to stand for 20 minutes. It was then concentrated in vacuo, the residue treated with saturated sodium bicarbonate solution (20 ml) and extracted with ethyl acetate.

The organic layer was washed with water, dried (Na₂SO₄) and concentrated to afford (625 mg, 70%) an oily residue i.e. the compound desired in this example which was crystallised as its oxalate from ethyl alcohol-dry ether, m.p. 190°C. The above compound on desired alklation or acylation at one or both of the hydroxy groups affords other products of compound I.

The present invention provides compounds of the formula I as described above for use in a method of medical treatment.

The invention also provides the use of compounds of the formula I in the manufacture of a medicament for the treatment of an estrogen-dependent condition such as breast cancer.

The compounds may be administered in the form of a pharmaceutical composition. The present invention provides a composition comprising a compound of formula I and a pharmaceutically acceptable carrier or diluent.

The compositions may be administered orally or parenterally, in a dosage of 1 to 1000 »g/kg, depending on factors such as the particular condition to be treated and the route of administration.

## Claims

1. A compound of the formula I 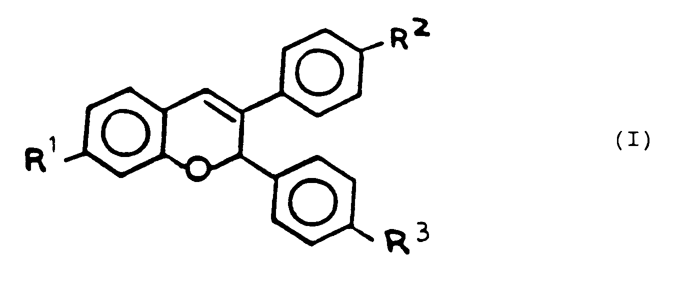 wherein R¹ and R², which may be the same or different, are each -H, -OH, alkoxy of 1 to 17 carbon atoms or alkoxycarbonyl of 2 to 18 carbon atoms, and R³ is

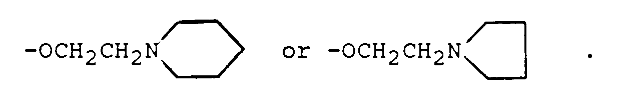

2. A compound as claimed in claim 1 in which R¹ and R² are each independently H, OH or C₁₋₄alkoxy.

3. A compound as claimed in claim 1 or 2 in which R¹ is H.

4. A compound as claimed in claim 1, 2 or 3 in which R³ is

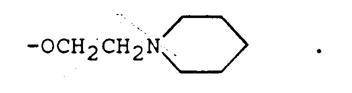

5. 2-[4-[2-(1-piperidino)ethoxy]phenyl]-3-[4-hydroxyphenyl]-2H-1-benzopyran.

6. 2-[4-[2-(1-piperidino)ethoxy]phenyl]-3-phenyl-7-methoxy-2H-1-benzopyran.

7. 2-[4-[2-(1-piperidino)ethoxy]phenyl]-3-[4-hydroxyphenyl]-7-hydroxy-2H-1-benzopyran.

8. A process for the preparation of a compound as claimed in any one of the preceding claims comprising reacting a compound of formula II 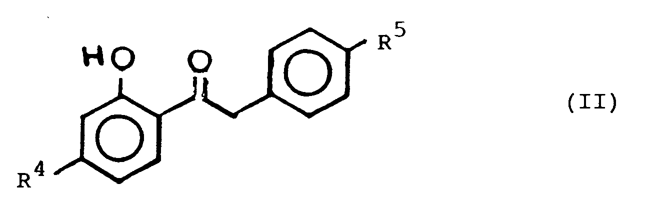 in which R⁴ and R⁵ are R¹ and R² respectively, or a protected hydroxy group, with 4-hydroxybenzaldehyde to produce a compound of the formula III 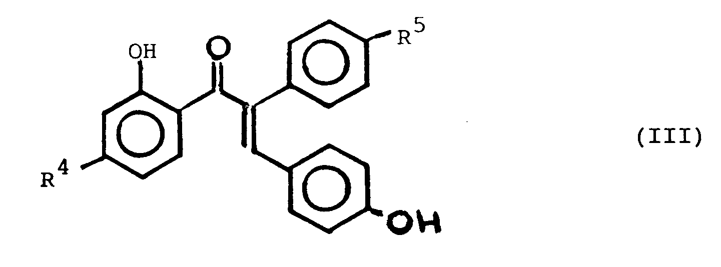 forming a compound of formula IV 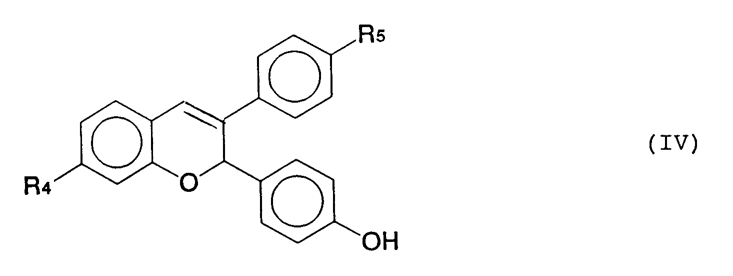 reacting this compound with a compound of formula V 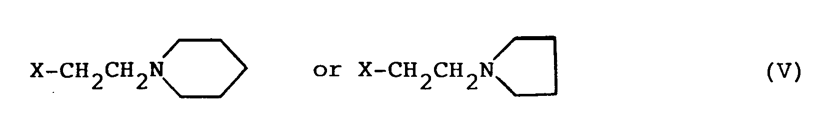 in which X is a halide to form a compound of formula VI 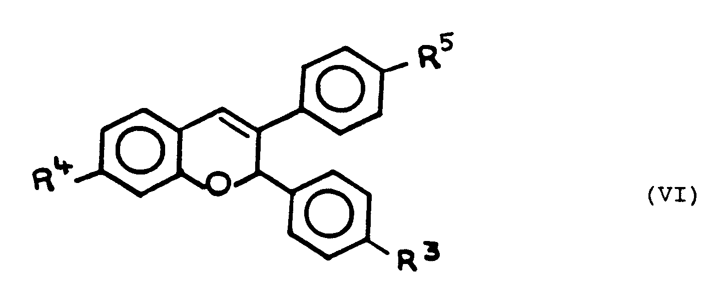 and, if necessary, deprotecting and acylating or alkylating R⁴ and R⁵.

9. A process according to claim 8 in which R⁴ is H and R⁵ is a protected hydroxy group.

10. A process according to claim 9 in which the unprotected starting compound of formula VII 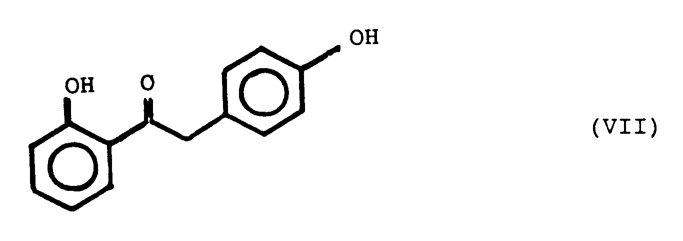 is produced by condensing phenyl with 4-methoxyphenylacetyl halide, and a Fries rearrangement in the presence of anhydrous aluminium chloride.

11. A process according to claim 8 in which both R⁴ and R⁵ are protected hydroxy groups.

12. A process according to any one of claims 8 to 11 in which the compound of formula II if protected is in the form of a tetrahydropyranyl ether.

13. A process according to claim 12 in which the protection is carried out by reacting the unprotected compound with dihydropyran in the presence of a sulphonic acid and an ether.

14. A process according to any one of claims 8 to 13 in which the conversion of compound II to compound III is carried out in the presence of an aromatic hydrocarbon solvent, containing a secondary and/or tertiary cyclic or open chain amino base.

15. A process according to any one of claims 8 to 14 in which compound II is converted into a mixture of compound III and compound VIII 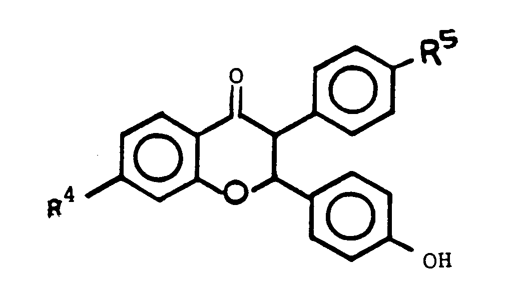 and compounds III and VIII are separated by chromatography.

16. A process according to any one of claims 8 to 15 in which conversion of compound III to compound IV is carried out by reduction and cyclodehydration.

17. A process according to claim 16 in which the reduction is effected by the addition of a hydride.

18. A process according to claim 17 in which the hydride is added in an alcoholic solvent, preferably in portions at 10 to 15 minute intervals for up to 15 hours.

19. A process according to any one of claims 16 to 18 in which the cyclodehydration is effected during work up of the product.

20. A process according to any one of claims 8 to 19 in which the reaction of compounds IV and V is carried out in the presence of a basic catalyst and a ketonic solvent.

21. A compound as claimed in any one of claims 1 to 7 for use in a method of medical treatment.

22. A composition comprising a compound as claimed in any one of claims 1 to 7 or 21 and a pharmaceutically acceptable carrier or diluent.

23. Use of a compound as claimed in any one of claims 1 to 7 in the manufacture of a medicament for the treatment of an estrogen-dependent condition.

## Patentansprüche

1. Verbindung der Formel I 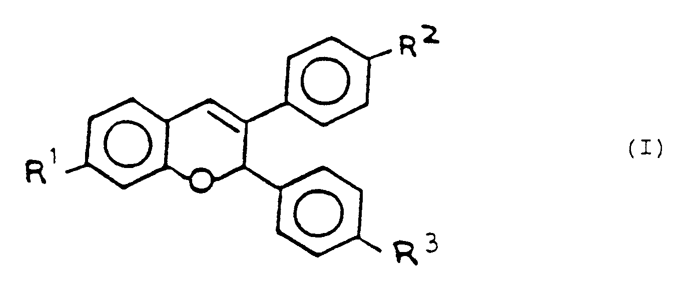 worin jeder der Reste R¹ und R², die gleich oder verschieden sein können, -H, -OH, Alkoxy mit 1 bis 17 Kohlenstoffatomen oder Alkoxycarbonyl mit 2 bis 18 Kohlenstoffatomen sind, und R³ 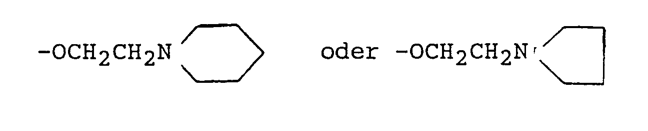 ist.

2. Verbindung nach Anspruch 1, dadurch gekennzeichnet, daß R¹ und R² unabhängig voneinander H, OH oder C₁₋₄-Alkoxy ist.

3. Verbindung nach Anspruch 1 oder 2, dadurch gekennzeichnet, daß R¹ H ist.

4. Verbindung nach Anspruch 1, 2 oder 3, dadurch gekennzeichnet, daß R³ 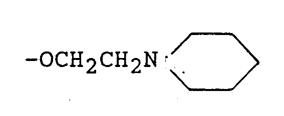 ist.

5. 2-[4-[2-(1-Piperidino)ethoxy]phenyl]-3-[4-hydroxyphenyl]-2H-1-benzopyran.

6. 2-[4-[2-(1-Piperidino)ethoxy]phenyl]-3-phenyl-7-methoxy-2H-1-benzopyran.

7. 2-[4-[2-(1-Piperidino)ethoxy]phenyl]-3-[4-hydroxyphenyl]-7-hydroxy-2H-1-benzopyran.

8. Verfahren zur Herstellung einer Verbindung nach einem der vorhergehenden Ansprüche, dadurch gekennzeichnet, daß man eine Verbindung der Formel II 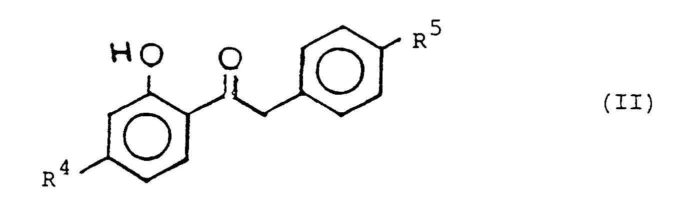 worin R⁴ und R⁵ R¹ bzw. R² darstellen, oder eine geschützte Hydroxygruppe mit 4-Hydroxybenzaldehyd unter Bildung einer Verbindung der Formel III umsetzt 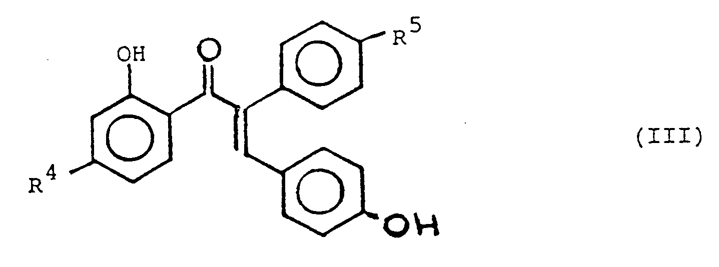 woraus eine Verbindung der Formel IV gebildet wird 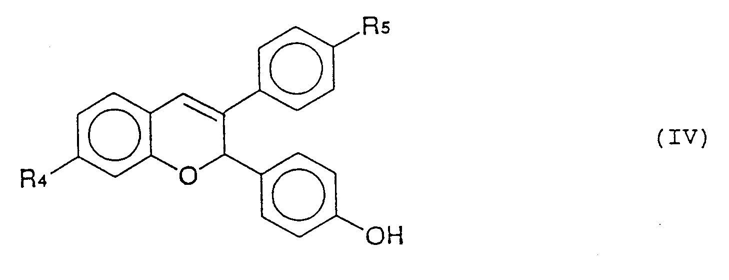 diese Verbindung mit einer Verbindung der Formel V 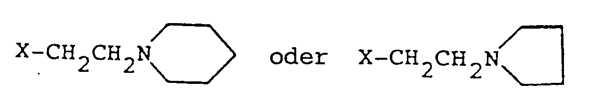 worin X ein Halogenid ist, unter Bildung einer Verbindung der Formel VI 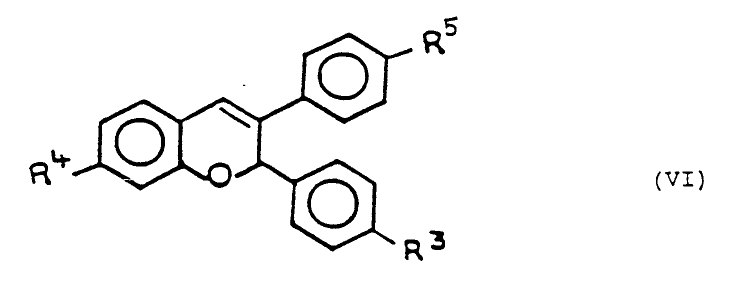 umsetzt, und wenn erforderlich, die Schutzgruppen entfernt und R⁴ und R⁵ acyliert oder alkyliert.

9. Verfahren nach Anspruch 8, dadurch gekennzeichnet, daß R⁴ H und R⁵ eine geschützte Hydroxygruppe ist.

10. Verfahren nach Anspruch 9, dadurch gekennzeichnet, daß die ungeschützte Ausgangsverbindung der Formel VII 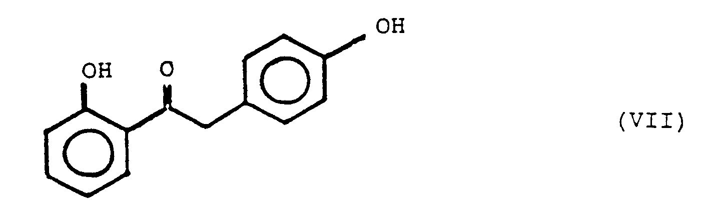 durch Kondensation von Phenol mit 4-Methoxyphenylacetylhalogenid und Fries-Umlagerung in Gegenwart von wasserfreiem Aluminiumchlorid hergestellt wird.

11. Verfahren nach Anspruch 8, dadurch gekennzeichnet, daß beide Reste R⁴ und R⁵ geschützte Hydroxygruppen sind.

12. Verfahren nach einem der Ansprüche 8 bis 11, dadurch gekennzeichnet, daß die Verbindung der Formel II, wenn geschützt, in Form eines Tetrahydropyranylethers vorliegt.

13. Verfahren nach Anspruch 12, dadurch gekennzeichnet, daß der Schutz durch Umsetzung der ungeschützten Verbindung mit Dihydropyran in Gegenwart einer Sulfonsäure und eines Ethers durchgeführt wird.

14. Verfahren nach einem der Ansprüche 8 bis 13, dadurch gekennzeichnet, daß die Überführung der Verbindung II in die Verbindung III in Gegenwart eines aromatischen Kohlenwasserstoff-Lösungsmittels, das eine sekundäre und/oder tertiäre cyclische oder offenkettige Aminobase enthält, durchgeführt wird.

15. Verfahren nach einem der Ansprüche 8 bis 14, dadurch gekennzeichnet, daß die Verbindung II in eine Mischung der Verbindung III und VIII 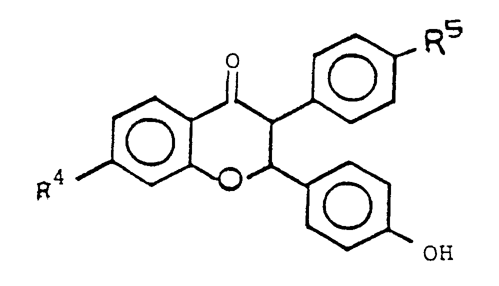 überführt wird, und die Verbindung III und VIII durch Chromatographie getrennt werden.

16. Verfahren nach einem der Ansprüche 8 bis 15, dadurch gekennzeichnet, daß die Überführung der Verbindung III in die Verbindung IV durch Reduktion und Cyclodehydrierung durchgeführt wird.

17. Verfahren nach Anspruch 16, dadurch gekennzeichnet, daß die Reduktion durch Zugabe eines Hydrids bewirkt wird.

18. Verfahren nach Anspruch 17, dadurch gekennzeichnet, daß das Hydrid in einem alkoholischen Lösungsmittel, vorzugsweise anteilig bei 10 bis 15 Minuten-Intervallen, während bis zu 15 Stunden zugegeben wird.

19. Verfahren nach einem der Ansprüche 16 bis 18, dadurch gekennzeichnet, daß die Cyclodehydrierung während der Aufarbeitung des Produktes bewirkt wird.

20. Verfahren nach einem der Ansprüche 8 bis 19, dadurch gekennzeichnet, daß die Umsetzung der Verbindung IV und V in Gegenwart eines basischen Katalysators und eines Keton-Lösungsmittels durchgeführt wird.

21. Verbindung nach einem der Ansprüche 1 bis 7 zur Verwendung in einem Verfahren zur medizinischen Behandlung.

22. Zusammensetzung, umfassend eine Verbindung nach einem der Ansprüche 1 bis 7 oder 21 und einen pharmazeutisch annehmbaren Träger oder Verdünner.

23. Verwendung einer Verbindung nach einem der Ansprüche 1 bis 7 zur Herstellung eines Arzneimittels zur Behandlung eines Östrogen-abhängigen Zustandes.

## Revendications

1. Composé de formule I 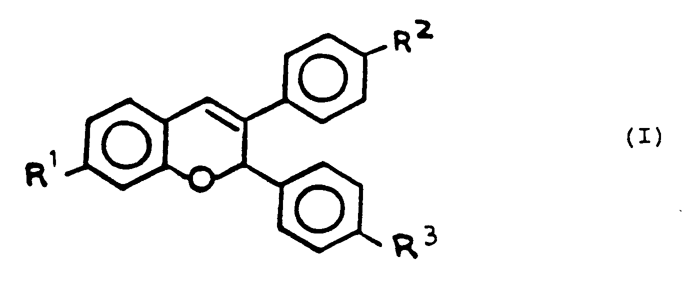 dans laquelle R¹ et R², qui peuvent être identiques ou différents, sont chacun -H, -OH, un alcoxy contenant 1 à 17 atomes de carbone ou un alcoxycarbonyle contenant 2 à 18 atomes de carbone et R³ est

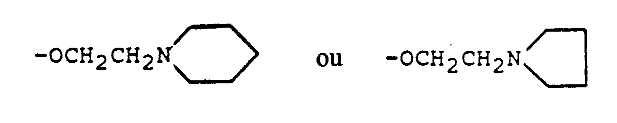

2. Composé selon la revendication 1, dans lequel R¹ et R² sont chacun, de façon indépendante l'un de l'autre, H, OH ou un alcoxy contenant 1 à 4 atomes de carbone.

3. Composé selon la revendication 1 ou 2, dans lequel R¹ est un H.

4. Composé selon la revendication 1, 2 ou 3, dans lequel R³ est

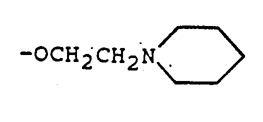

5. 2-[4-[2-(1-pipéridino)éthoxy]phényl]-3-[4-hydroxyphényl]-2H-1-benzopyranne.

6. 2-[4-[2-(1-pipéridino)éthoxy]phényl]-3-phényl-7-méthoxy-2H-1-benzopyranne.

7. 2-[4-[2-(1-pipéridino)éthoxy]phényl]-3-[4-hydroxyphényl]-7-hydroxy-2H-1-benzopyranne.

8. Procédé pour la préparation d'un composé selon une quelconque des revendications qui précèdent constitué par la réaction d'un composé de formule II 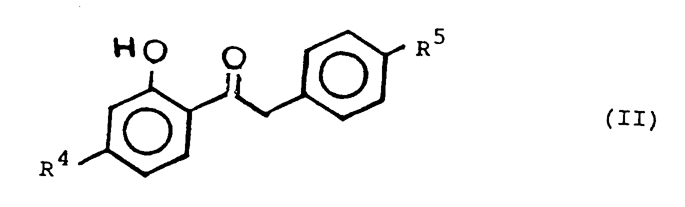 dans laquelle R⁴ et R⁵ sont respectivement R¹ et R² ou un groupe hydroxyle protégé, avec le 4-hydroxybenzaldéhyde pour produire un composé de formule III 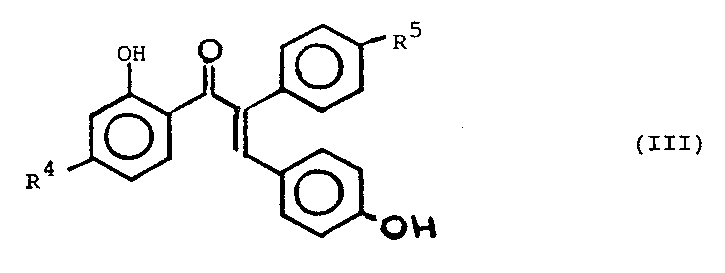 la formation d'un composé de formule IV 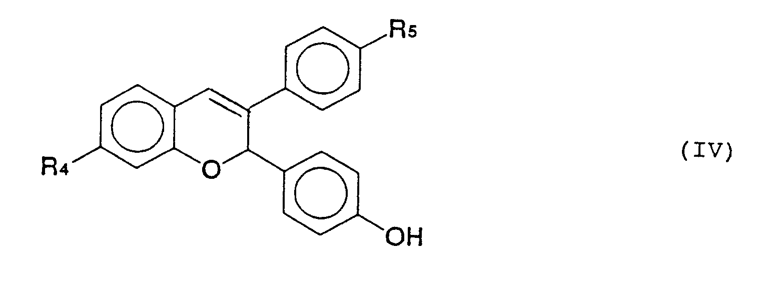 la réaction de ce composé avec un composé de formule V 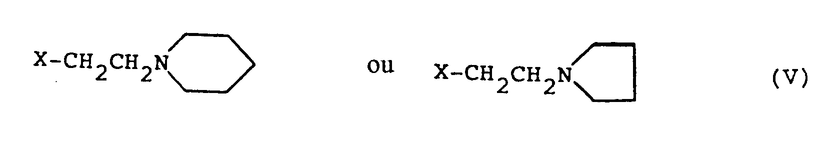 où X est un halogénure pour former un composé de formule VI 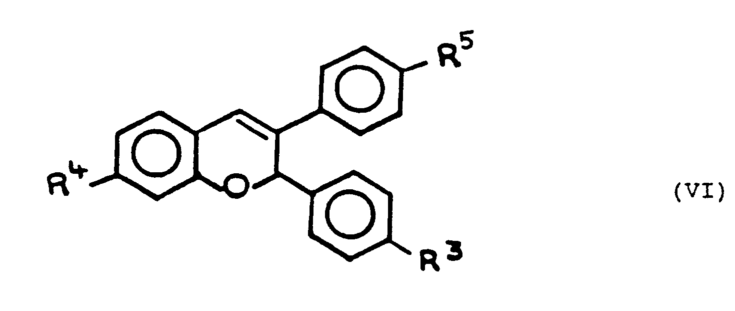 et, si besoin est, la déprotection et l'acylation ou l'alkylation de R⁴ et R⁵.

9. Procédé selon la revendication 8, dans lequel R⁴ est H et R⁵ est un groupe hydroxyle protégé.

10. Procédé selon la revendication 9, dans lequel le composé de départ non protégé de formule VII 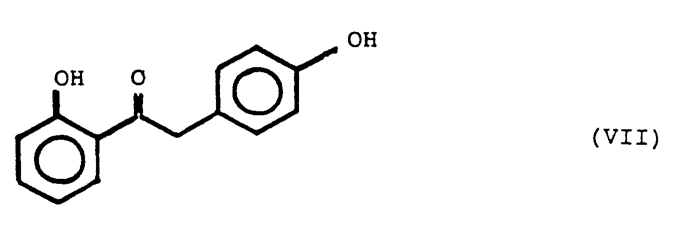 est produit par la condensation d'un phényle avec un halogénure de 4-méthoxyphénylacétyle et par une transposition de Fries en présence de chlorure d'aluminium anhydre.

11. Procédé selon la revendication 8, dans lequel R⁴ et R⁵ sont tous deux des groupes hydroxyle protégés.

12. Procédé selon une quelconque des revendication 8 à 11, dans lequel le composé de formule II, s'il est protégé, est sous la forme d'un éther de tétrahydropyrannyle.

13. Procédé selon la revendication 12, dans lequel la protection est réalisée en faisant réagir le composé non protégé avec le dihydropyranne en présence d'un acide sulfonique et d'un éther.

14. Procédé selon une quelconque des revendication 8 à 13, dans lequel la conversion du composé II en composé III est réalisée en présence d'un solvant hydrocarbure aromatique contenant une base aminée secondaire et/ou tertiaire, cyclique ou à chaîne ouverte.

15. Procédé selon une quelconque des revendication 8 à 14, dans lequel le composé II est converti en un mélange de composé III et composé VIII 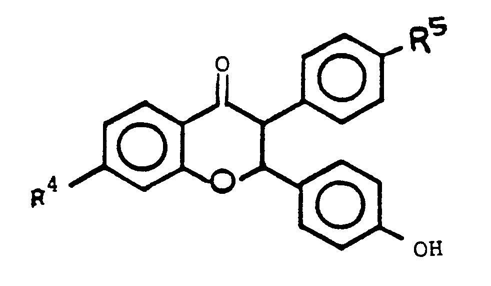 et dans lequel les composés III et VIII sont séparés par chromatographie.

16. Procédé selon une quelconque des revendication 8 à 15 dans lequel la conversion du composé III en composé IV est réalisée par réduction et cyclodéshydratation.

17. Procédé selon la revendication 16, dans lequel la réduction est effectuée par addition d'un hydrure.

18. Procédé selon la revendication 17, dans lequel l'hydrure est ajouté dans un solvant alcoolique, de préférence par fractions à des intervalles de 10 à 15 minutes, pendant jusqu'à 15 heures.

19. Procédé selon une quelconque des revendications 16 à 18, dans lequel la cyclodéshydratation est effectuée pendant le traitement du produit.

20. Procédé selon une quelconque des revendications 8 à 19, dans lequel la réaction des composés IV et V est réalisée en présence d'un catalyseur basique et d'un solvant cétonique.

21. Composé selon une quelconque des revendications 1 à 7 pour son utilisation dans une méthode de traitement médical.

22. Composition constituée par un composé selon une quelconque des revendications 1 à 7 ou 21 et un excipient ou un diluant pharmaceutiquement acceptable.

23. Utilisation d'un composé selon une quelconque des revendications 1 à 7 dans la fabrication d'un médicament pour le traitement d'une pathologie liée aux oestrogènes.
